# EUROPEAN PATENT APPLICATION

(11) **EP 1 213 019 A2**
(43) Date of publication of application: **12.06.2002**
(21) Application number: 01128074.0
(22) Date of filing: 27.11.2001
(51) Int. Cl.: A61K 31/54, A61P 11/08

(54) **Method for treating chronic obstructive pulmonary disease**

(30) Priority: 07.12.2000 US 251998 P
(71) Applicant: WARNER-LAMBERT COMPANY, Morris Plains New Jersey 07950 (US)
(72) Inventor: Keller, Laurence Harvey, Ann Arbor, Michigan 48103 (US); Pressler, Milton Lethan, Saline, Michigan 48176 (US)
(74) Representative: Tesch, Rudolf, Dr.

(57) **Abstract**

Chronic obstructive pulmonary disease is treated with a selective ET_{A} receptor antagonist such as 4-(7-ethyl-1,3-benzodioxol-5-yl)-2-[2-trifluoromethyl)phenyl]-2H-1,2-benzothiazine-3-carboxylic acid 1,1-dioxide or a pharmaceutically acceptable salt thereof.

## Description

### FIELD OF THE INVENTION

This invention relates to a method for treating chronic obstructive pulmonary disease in a mammal by administering a highly selective endothelin Subtype A receptor (ET_{A}) antagonist.

### BACKGROUND OF THE INVENTION

Chronic obstructive pulmonary disease (COPD) is a syndrome characterized by a variety of symptoms such as hacking cough, brochoconstrmucus hypersecretion, breathlessness, and chest tightness. Patients having COPD are highly susceptible to pulmonary infections such as colds and viruses, and the condition is also characterized by episodes of increased severity of symptoms of these conditions and hospitalization. COPD is typically a disease of the more elderly population, and individuals who smoke are at an even higher risk for developing COPD than non smokers. In fact, COPD often is a consequence of smoking. It also is caused by air pollution. As COPD becomes more advanced, it can have a profound adverse effect on the quality of a patient's life, for example, with respect to mobility and the ability to carry out everyday tasks. Left untreated, COPD is progressive and is life threatening, and claims about three million lives annually.

There are currently few treatments for patients suffering from COPD. Such patients generally are given antibiotics to treat acute exacerbations of the disease, although it is not always clear that the condition is driven by an underlying bacterial infection. Most patients suffering from COPD are given constant bronchodilator therapy in an effort to keep the airways open as much as possible. Unlike asthma, however, only limited bronchodilation can be achieved in patients suffering from COPD, because the obstruction of the airway is not attributable to just contraction of the muscles in the airway walls.

The most common treatment for COPD patients has been inhaled steroidial anti-inflammatory agents. However, recent data have established that the inflammatory processes in COPD are much different from those in asthma, and that steroids really are of little use to most COPD patients.

The prevalence of COPD is rising due to increased air pollution, the aging population, and because COPD is particularly serious in older patients. There is thus a significant need to develop new agents that are effective in treating this disease and that can be conveniently administered. This is especially critical since there are currently few effective treatments available. An object of this invention is to provide a new method for treating COPD comprising administering a compound that selectively inhibits a peptide known as an endothelin ET_{A} receptor.

### SUMMARY OF THE INVENTION

This invention provides a method for treating COPD in a patient in need of treatment comprising administering an effective amount of a compound that selectively inhibits the endothelin ET_{A} receptor. The invention more particularly provides a method of treating COPD comprising administering the compound 4-(7-ethyl-1,3-ben zodioxol-5-yl)-2-[2-(trifluoromethyl)phenyl]-2H-1,2-benzothiazine-3-carboxylic acid 1,1-dioxide or a pharmaceutically acceptable salt thereof. In a preferred embodiment, the compound is administered as the monopotassium salt. This salt form is referred to as "CI-1034." CI-1034 has the following Formula I, and its physical characteristics are summarized below:

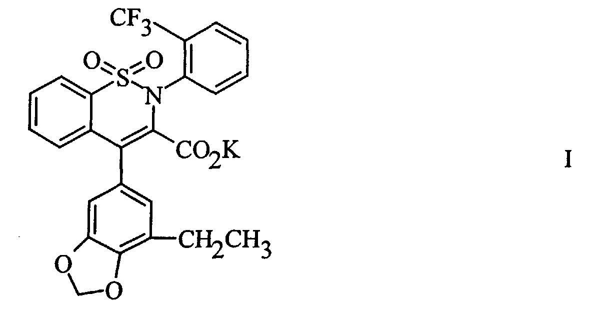

### DETAILED DESCRIPTION OF THE INVENTION

The compounds required to practice the method of this invention are selective endothelin ET_{A} antagonists. The method is preferably carried out by administering a compound that is described in US Patent No. 5,599,881 and in WO 99/12916 (both of which are incorporated herein by reference). The compound is described as an endothelin antagonist, and as such is said to be useful for treating essential renovascular malignant and pulmonary hypertension, cerebral infarction, cerebral ischemia, congestive heart failure, and subarachnoid hemorrhage. The compound is a nonpeptide selective endothelin ET_{A} receptor antagonist currently undergoing clinical development for treatment of pulmonary hypertension and pulmonary hypertension in congestive heart failure. There is no mention or suggestion that the compound would be useful to treat COPD.

The following definitions apply to the terms used in this Specification and in the appended claims.

"COPD" means chronic obstructive pulmonary disease.

"Endothelin" is the name of a peptide that plays a role in regulating vascular resistance and is a factor in the pathogenesis of human pulmonary hypertension and congestive heart failure. Patients with these conditions have increased levels of circulating endothelin peptide. There are two main endothelin subtype receptors, namely the ET_{A} and the ET_{B} subtype receptors. The ETA endothelin receptor subtype is found on vascular smooth muscle cells and cardiocytes, and these receptors mediate vasoconstriction and cellular proliferation. The endothelin ET_{B} receptors are located predominantly on endothelial cells, but are located on smooth muscle cells as well. The endothelin ET_{B} receptor is associated with other disease states, but also mediates vasodilation via production of nitric oxide and prostacyclin. Patients having COPD have elevated levels of endothelin which activates ET_{A} receptors, thereby causing uncontrolled vasoconstriction and vascular proliferation.

A "selective inhibitor of the endothelin ET_{A} receptor," also referred to as "a selective ET_{A} receptor antagonist" is a compound that inhibits the endothelin ET_{A} receptor at least 1000 times more effectively than it inhibits the endothelin ET_{B} receptor in standard in vitro or in vivo assays that measure endothelin antagonism, and thus has an ET_{A} to ET_{B} ratio (of inhibition) of at least 1000. Typical selective ET_{A} receptor antagonists include CI-1034, enrasentan, and atrasentan.

The term "patient" means a mammal, particularly a human, having COPD or showing symptoms associated with COPD. A patient can also be an animal such as a dog, cat, horse, or cow.

All that is required to practice the method of this invention is to administer an effective dose of a selective ET_{A} antagonist to a patient having COPD and in need of treatment. In a preferred embodiment the ET_{A} antagonist is CI-1034. The term "effective dose" means that amount of the selective ET_{A} antagonist required to ameliorate the symptoms manifested by the COPD disease state in a particular patient. An effective dose typically will be that amount of selective ET_{A} antagonist that when administered to a patient produces a blood plasma concentration ranging from about 0.01 to about 1.0 µg per mL. Effective doses will typically be from about 1.0 mg/kg of patient body weight to about 500 mg/kg. In a preferred embodiment, the typical dose will be from about 1.0 mg/kg to about 100 mg/kg. An even more preferred dose will be from about 1.0 mg/kg to about 20 mg/kg, administered from about 1 to about 4 times per day, or more often as determined by the attending medical practitioner.

In practicing the method of this invention, a selective ET_{A} receptor antagonist such as CI-1034 can be prepared as pharmaceutical compositions suitable for oral or parenteral administration, as well as transdermal and intranasal dosing. In a preferred embodiment, CI-1034 is formulated with common excipients and carriers for oral administration in the form of tablets, capsules, syrups, solutions, cachets, buccal seals, and the like. The compound can also be prepared as a parenteral composition for injection, for example, intramuscularly, intracutaneously, subcutaneously, intraduodenally, or intraperitoneally. The compound is generally dissolved in isotonic saline or 5% aqueous glucose or other suitable diluent. CI-1034 can additionally be administered by inhalation, for instance intranasally. In addition to CI-1034, other selective ET_{A} receptor antagonists and salt forms of the above compounds can be prepared and utilized in the same manner, for example as fully described in US 5,599,811, which is incorporated herein by reference. Other salt forms of the compound of Formula I include the sodium and calcium salts, as well as organic salts such as triethylamine and pyridine.

The biological activities of the compounds of Formula I have been evaluated in a number of in vitro and in vivo assays. CI-1034 has been shown to be a highly selective antagonist of the ET_{A} subtype receptor, and is surprisingly more potent and selective than other known endothelin antagonists. For example, CI-1034 was compared with several known endothelin receptor antagonists currently undergoing clinical development for treating congestive heart failure, stroke, and hypertension. The results of the comparisons are presented in TABLE 1 below.

**TABLE 1.**

| Receptor Inhibition (nM) | | | |
|---|---|---|---|
| | Human ET_{A} | Human ET_{B} | A/B Ratio |
| CI-1034 | 0.6 | 1600 | 2700 |
| Bosentan | 4.7 | 95 | 20 |
| Enrasentan | 0.1 | 111 | 1110 |
| Darusentan | 1.4 | 184 | 131 |
| Atrasentan | 0.07 | 139 | 2000 |

The foregoing results establish that CI-1034 is highly selective at inhibiting the ET_{A} receptor, and as such is ideally suited to treating COPD according to this invention.

The following detailed examples further establish the biological spectrum of activities exhibited by compounds of Formula I.

### EXAMPLE 1

### Characteristics of CI-1034

- Chemical Name:: 4-(7-Ethyl-1,3-benzodioxol-5-yl)-2-[2-(trifluoromethyl)phenyl]-2H-1,2-benzothiazine-3-carboxylic acid 1,1-dioxide monopotassium salt
- Code Names:: CI-1034, PD 0180988-0016
- Molecular Formula:: C₂₅H₁₇F₃NO₆S·K
- Molecular Weight:: 555.57 monopotassium salt, 517.48 free acid
- Appearance:: Pink or white to tan solid
- Dissociation Constant:: The apparent pKa determined by potentiometric titration in water is 3.9 corresponding to the carboxylic acid functional group.
Solubility: CI-1034 is freely soluble in water and insoluble in 0.1N HCl. The compound acts as a base, raising the pH of the water to a measured value of 8.9 for a 100 mg/mL solution. CI-1034 is very slightly soluble in 0.05 M pH 4 acetate buffer and 0.05 M pH 7.4 potassium phosphate buffer. In 0.5 M pH 7.4 potassium phosphate buffer, CI-1034 dissolves rapidly, reaching a concentration of approximately 13 mg/mL, before decreasing to approximately 0.8 mg/mL after 24 hours.
CI-1034 is insoluble in hexane and isopropanol. The compound is very slightly soluble in chloroform and dichloromethane. CI-1034 is slightly soluble in acetonitrile and tetrahydrofuran, sparingly soluble in ethanol, and freely soluble in dimethylsulfoxide and methanol.
Partition Coefficient: The octanol-aqueous partition coefficient at various pH values was determined by the shake-flask method using HPLC for measurement of the amount of compound in each phase. The results are listed in TABLE 2.

**TABLE 2.**

| Partition Coefficient Values for CI-1034 | |
|---|---|
| Aqueous Medium | Log D |
| 0.1N Hydrochloric Acid | 3.24 |
| pH 4, 0.05 M Sodium Acetate Buffer | 2.93 |
| pH 7.4, 0.05 M Potassium Phosphate Buffer | 0.56 |

Thermal Properties: CI-1034 is thermally stable up to approximately 300°C, where it begins to melt with decomposition.
Hygroscopicity: CI-1034 is moderately hygroscopic.
Stability: CI-1034 is stable when heated in acid, base, or water when protected from light. No significant degradation occurred in a sample exposed to hydrogen peroxide. In contrast, samples exposed to simulated sunlight showed degradation.
CI-1034 was stable for 4 weeks when stored at 80°C or 40°C and 70% relative humidity. CI-1034 does not need special storage requirements, other than the usual protection from light.

### EXAMPLE 2

### Preclinical Evaluation of Efficacy

CI-1034 is orally active and is intended for oral administration clinically in a preferred embodiment.

CI-1034 has demonstrated efficacy in blocking exogenously applied ET-1 in cell culture, in isolated vasculature, and in intact animals. In addition, CI-1034 has been shown to be efficacious in pathologic models of acute and chronic pulmonary hypertension induced by hypoxia.

### Blockade of ET_{A} and ET_{B} Receptor Signaling in Cell Culture

Cell cultures of human pulmonary artery smooth muscle cells (hPASMC) expressing predominantly ET_{A} receptors (83%) were used to determine the effectiveness of CI-1034 in blocking human ET_{A} receptor signaling. When intact hPASMCs were loaded with Calcium Green-1 dye and exposed to ET-1 (100 nM), a fluorescent signal was produced that was comparable to that observed with a Ca²⁺ ionophore (positive control). In contrast, the ET_{B} selective agonist sarafotoxin-6c (S6c) did not evoke a fluorescent signal at a concentration (100 nM) known to maximally activate ET_{B} receptors. These results indicate that the Ca²⁺ transient produced by ET-1 in these hPASMCs was mediated predominantly by ET_{A} receptors. Pretreatment of the hPASMCs with CI-1034 produced a concentration-dependent inhibition of the ET-1 (100 nM) response; the concentration that produced half-maximal inhibition (IC₅₀) was 0.22 nM.

To determine the effectiveness of CI-1034 in blocking ET_{B} receptor signaling, [³H] arachidonic acid (AA) was incorporated into membranes of Chinese hamster ovary (CHO) cells transfected with recombinant human ET_{B} receptors. ET_{B} activation with S6c (100 nM) causes maximal release of [³H]AA from the cell membrane into the culture medium. Pretreatment of CHO cell with CI-1034 produced a concentration-dependent inhibition of the S6c mediated [³H]AA release, and the concentration that produced half-maximal inhibition (IC₅₀) was 2200 nM. Collectively, these results indicate CI-1034 is approximately 10,000-fold selective in blocking human ET_{A} receptor signaling versus human ET_{B} receptor signaling.

### EXAMPLE 3

### Blockade of ET-1 Induced Contraction in Isolated Rabbit Vascular Tissue

In rabbit femoral artery vasculature, the receptor responsible for mediating the contractile activity of ET-1 appears to be predominantly the ET_{A} receptor subtype, whereas in the rabbit pulmonary artery the contractile activity of ET-1 appears to be mediated by both ET_{A} and ET_{B} receptors. These differences in receptor expression make it possible to determine the relative potency of CI-1034 for blocking ET_{A} and ET_{B} receptor signaling in vascular tissue.

In rabbit femoral and pulmonary arteries, ET-1 produced concentration-dependent increases in contractile tension starting at 1 nM. Pretreatment of femoral arteries with CI-1034 resulted in rightward shifts in the ET-1 concentration response curves. At the concentration of 1 µM, CI-1034 produced approximately 30-fold rightward shift in the ET-1 response curve resulting in a K_{B} (inhibitor constant of antagonist) value of 25 nM. In contrast, 100 µM CI-1034 produced approximately a 10-fold shift in the ET-1 concentration-response curve in rabbit pulmonary artery resulting in a K_{B} value of 9720 nM. Since the ET-1 response in the rabbit femoral artery is predominantly mediated by ET_{A} receptor, the much higher K_{B} in the rabbit pulmonary artery reflects the concentration of CI-1034 necessary to block both ET_{A} and ET_{B} receptor signaling. Based on these findings CI-1034 is 389-fold more potent at blocking ET_{A} receptor signaling versus ET_{B} receptor signaling in isolated rabbit vasculature.

Schild analysis of the ET- 1 concentration-response curves in rabbit femoral arteries indicates that CI-1034 produced parallel rightward shift in a manner consistent with ET_{A} competitive antagonism. The slopes of the regression line approximated unity (0.88, r = 0.99) and ET-1 induced maximal contraction was not affected by CI-1034. The pA2 (-log K_{B}) value of 7.6 was based on the regression line x-intercept. These results indicate CI-1034 competitively antagonized the contractile activity of ET-1 in rabbit femoral artery.

### EXAMPLE 4

### Blockade of bET-1 Induced Pressor Responses in Rats

Rats were administered vehicle (water) or CI-1034 (30 mg/kg) by oral gavage 24 hours prior to a big-endothelin-1 (bET-1) (precursor to ET-1) (1 nmol/kg, IV bolus) challenge; the subsequent pressor response is mediated principally by ET_{A} receptors. Vehicle-treated rats responded to the bET-1 challenge with peak increases in mean arterial pressure (MAP) of 58 ± 2 mm Hg (N = 7); baseline MAP was 82 ± 5 mm Hg. In comparison, rats pretreated with CI-1034 responded to the bET-1 challenge with a peak pressor response of 35 ± 7 mm Hg, which represented a 39% inhibition of the bET-1 pressor response compared to vehicle-treated rats (p <0.05). The CI-1034 plasma concentration 24 hours after the 30-mg/kg dose averaged 0.16 ± 0.01 µg/mL.

### EXAMPLE 5

### Effects of CI-1034 in Blocking Pulmonary Hypertension in Response to Acute Hypoxia

Exposure to hypoxia produces an acute pulmonary hypertensive response in the rat. This hypertensive response is accompanied by an increase in circulating ET-1 levels suggesting a causative role. The hypothesis has since been tested through blockade of the hypertensive response with several ET antagonists. The antihypertensive dose of CI-1034 was determined in response to acute hypoxia. Rats instrumented with pulmonary artery catheters received oral doses of vehicle (1 mL/kg, water) or CI-1034 (0.3 or 3.0 mg/kg, oral [PO]) 30 minutes before exposure to hypoxia (10% O₂). In vehicle-treated rats 4 hours of hypoxia significantly increased mean pulmonary artery pressure (MPAP) from 13 ± 0 to 29 ± 1 mm Hg (N = 3). CI-1034 pretreatment significantly reduced the peak hypertensive response at 4 hours by 34% ± 1% and 70% ± 3% at 0.3 and 3.0 mg/kg, respectively. The dose of CI-1034 calculated to inhibit the acute pulmonary hypertensive response (relative to the hypoxic control group) by 50% (ED₅₀) is 0.8 mg/kg PO.

A simple Emax model suggested a relationship between CI-1034 plasma concentration and pulmonary antihypertensive efficacy in this acute hypoxia model. Heparinized plasma samples were collected 10 minutes after the final MPAP measurement was made. The predicted maximal antihypertensive effect is 81% ± 23%, and the concentration of CI-1034 predicted to produce half-maximal effect is 0.03 ± 0.03 µg/mL.

### EXAMPLE 6

### Effects of CI-1034 on Pulmonary Hypertension and Right Ventricular (RV) Hypertrophy in Rats Exposed to Chronic Hypoxia

Exposure to chronic hypoxia produces a sustained and progressive increase in pulmonary artery pressure in rats. Consequently, there is a pressure overload on the right ventricle (RV) and progressive RV hypertrophy. ET-1 circulating levels are acutely increased in response to hypoxia and remain elevated after chronic exposure. These observations suggest that ET-1 contributes to the initiation and progression of chronic pulmonary hypertension and RV hypertrophy through its vasoconstrictor and mitogenic activities. The hypothesis has since been tested with the demonstration that ET antagonists are effective in preventing (treatment from the start of hypoxia) and attenuating (treatment after pulmonary hypertension and RV hypertrophy has been established) these progressive effects of chronic hypoxia.

An intervention protocol was used to determine the dose/plasma concentration of CI-1034 necessary to attenuate the progression of pulmonary hypertension and RV hypertrophy in response to chronic hypoxia. Rats were randomized to CI-1034 treatment (25, 50, or 100 mg/kg/day), administered in the diet (ground rat chow), or diet alone (hypoxic control) after 10 days of exposure to hypoxia (10% O₂). Rats were exposed to hypoxia for an additional 10 days during treatment (20 days total). A separate group of age-matched rats treated with diet alone (20 days) and no exposure to hypoxia served as normoxic controls. Blood samples, pulmonary artery pressures, and cardiac right and left ventricles were collected at the end of the 20-day protocol.

Rats exposed to 20 days of hypoxia had MPAP of 43 ± 2 mm Hg which averaged 30 mm Hg higher than normal (13 ± 0 mm Hg). The MPAPs in rats treated with CI-1034 were lower than hypoxic controls, the effects of CI-1034 were dose-related. The average MPAPs from hypoxic rats treated with CI-1034 at the daily dose level of 25, 50, and 100 mg/kg were 37 ± 2, 33 ± 0, and 29 ± 2 mm Hg, respectively.

To estimate an effective CI-1034 plasma concentration in chronic pulmonary hypertension, a simple Emax model was used to predict maximal CI-1034 antihypertensive effect and the concentration of CI-1034 that would produce a half-maximal antihypertensive effect. Heparinized plasma samples were collected approximately 1 hour before MPAP was measured. The predicted maximal antihypertensive effect of CI-1034 in this model is 60% ± 18%, and the concentration of CI-1034 predicted to produce half-maximal effect is 0.32 ± 0.25 µg/mL. The model suggests a relationship between plasma concentration and antihypertensive effect.

The right ventricular hypertrophic response to hypoxia was estimated by comparing the ratio of right ventricular free wall weight to left ventricle plus septum weight (RV/LV+S) between hypoxic and normoxic control groups. Using this index, rats exposed to hypoxia had right ventricular free walls that were more than twice control (0.57 ± 0.03 versus 0.24 ± 0.1). This right ventricular hypertrophic response to hypoxia was less in rats treated with CI-1034 at the daily doses of 50 and 100 mg/kg with (RV/LV+S) ratios of 0.42 ± 0.02 and 0.40 ± 0.02, respectively.

### EXAMPLE 7

### Mechanism of Action

Competition and saturation binding studies show that CI-1034 is a potent, competitive and selective inhibitor of [¹²⁵I]ET-1 binding to human recombinant ET_{A} receptors.

### Selective ET_{A} Receptor Binding

Membranes prepared from either Ltk-cells (mouse fibroblast, thymidine kinase deficient, cell line) or CHO cells that were transfected to express recombinant human ET_{A} or ET_{B} receptors, respectively, were used to determine the potency and selectivity of CI-1034 at inhibiting [¹²⁵I]ET-1 binding to ET_{A} receptors or [¹²⁵I]ET-3 binding to ET_{B} receptors.

CI-1034 effectively inhibited [¹²⁵I]ET-1 binding to ET_{A} receptors at subnanomolar concentrations (IC₅₀ = 0.6 nM). In comparison, micromolar concentrations of CI-1034 were required to inhibit [¹²⁵I]ET-3 binding to ET_{B} receptors (IC₅₀ = 1600 nM). Collectively, these results indicate that CI-1034 is about 2600-fold selective for human ET_{A} receptors.

### Saturation Binding Results

Membranes prepared from Ltk-cells that were transfected to express recombinant human ET_{A} receptors were used to examine the competitive nature of CI-1034 binding to ET_{A} receptors.

CI-1034 reduced the apparent Kd, the equilibrium dissociation constant, of [¹²⁵I]ET-1 binding without significantly affecting the Bₘₐₓ (maximal [¹²⁵I]ET-1 binding). These results are consistent with CI-1034 acting as a competitive inhibitor of ET-1 binding to ET_{A} receptors. Scatchard analysis of these data produced a Kᵢ (inhibitor constant of unlabeled antagonist) value of 0.51 nM for CI-1034.

### EXAMPLE 8

### Comparison of Human, Dog, and Rat CI-1034 ET_{A} and ET_{B} Receptor Binding

CI-1034 inhibited [¹²⁵I]ET-1 binding to dog and rat ET_{A} receptors at concentrations that were 3- to 5-fold higher than those required to inhibit [¹²⁵I]ET-1 binding to cloned human ET_{A} receptors. In contrast, CI-1034 was a more potent inhibitor of [¹²⁵I]ET-3 binding to dog and rat ET_{B} receptors compared to cloned human ET_{B} receptors. Collectively, these results indicate that CI-1034 is less ET_{A} selective in dog and rat compared to human. Relative binding activities are given below in TABLE 3.

**TABLE 3.**

| Comparison of Human, Dog, and Rat CI-1034 ET_{A} and ET_{B} Receptor Binding | | | |
|---|---|---|---|
| Species | ET_{A}, IC₅₀ (nM) | Species | ET_{B}, IC₅₀ (nM) |
| Human^{a} | 0.6 | Human^{b} | 1600 |
| Rat^{c} | 3.3 | Rat^{d} | 230 |
| Dog^{e} | 2.0 | Dog^{d} | 329 |

| | | | |
|---|---|---|---|
| ^{a} Membranes prepared from Ltk-cells with cloned human ETA receptors. | | | |
| ^{b} Membranes prepared from CHO cells with cloned human ETB receptors. | | | |
| ^{c} Membranes prepared from rat A 10 cells. | | | |
| ^{d} Membranes prepared from cerebellum. | | | |
| ^{e} Membranes prepared from dog cardiac ventricle. | | | |

### Estimated Effective Plasma Concentration in Humans

Based on the plasma concentration-effect relations for the antihypertensive activity of CI-1034 in the acute and chronic models of hypoxia induced pulmonary hypertension, the estimated effective plasma concentration range in humans is between 0.03 and 0.32 µg/mL.

In vitro binding studies in cultured cell membranes established that CI-1034 selectively binds to the human ET_{A} receptor in a competitive manner. Further studies in cultured cells have shown that CI-1034 is a potent and selective antagonist of ET-1 signaling via the human ET_{A} receptor. Selective ETA antagonism was also demonstrated at the tissue level in isolated vasculature where CI-1034 selectively blocked ET-1 induced contractile activity mediated through the ET_{A} receptor. In vivo blockade and oral duration of action were established by showing that CI-1034 effectively blocked the systemic pressor activity of a bET-1 challenge 24 hours after a single oral dose of 30 mg/kg. The ability of oral CI-1034 to block endogenously produced ET-1 was demonstrated by dose-dependent blockade of acute, hypoxia-induced pulmonary hypertension in the rat. This observation was extended to show that CI-1034 could moderate the progression of established pulmonary hypertension and right ventricular hypertrophy with chronic treatment. Based on the CI-1034 concentration-pulmonary antihypertensive effect relationship, an effective human plasma concentration range will be about 0.03 to about 0.32 µg/mL.

CI-1034 is absorbed from the gastrointestinal tract with PO bioavailability ranging from a low of approximately 10% in monkeys to a high of approximately 100% in dogs. CI-1034 radioequivalents slowly distribute into tissues with highest concentrations in liver and blood, indicating CI-1034 is not widely distributed. CI-1034 is highly bound to plasma proteins. Plasma concentrations increase approximately proportional to dose up to 100 mg/kg in dog. Incubations with rat, dog, monkey, and human hepatocyte preparations showed qualitatively similar metabolic profiles. Biliary elimination is the principal route of excretion of CI-1034-derived radioactivity.

### EXAMPLE 9

### Pulmonary Effects in Dogs

The potential effects of CI-1034 on pulmonary function were assessed in anesthetized dogs. Originally, dogs were to receive intravenous doses of 0.9% NaCI or CI-1034 at 40 or 180 mg/kg using a Latin-square design. Respiratory arrest was noted in one animal at 180 mg/kg within 5 minutes postdose. This animal subsequently recovered from anesthesia, had severe clinical signs on Day 1, and was found dead on Day 3. It was not known if respiratory arrest was a direct or secondary pulmonary effect, but this dose was clearly intolerable. No effects were apparent at 40 mg/kg; data from the only animal at this dose was not analyzed statistically. To assess pulmonary function at clinically tolerable doses, additional animals were given vehicle or CI-1034 at 30 and 60 mg/kg. Each animal received each treatment and served as its own control, with 7 days between treatments. Based on plasma CI-1034 concentrations 5 minutes postdose, exposures at these doses were at least 940 times the projected human therapeutic Cmax of 0.32 µg/mL. Pulmonary function parameters were evaluated for 60 minutes after each dose, and included minute volume, respiratory rate, resistance, compliance, peak inspiratory and expiratory volumes, and tidal volume. Indirect blood pressure measurements were made periodically throughout the pulmonary data collection period since endothelin receptor antagonists are known to induce hypotension.

CI-1034 had no effect on minute volume, pulmonary resistance, or pulmonary compliance at 30 or 60 mg/kg, and there was no significant effect on blood pressure. Plasma CI-1034 concentrations increased proportionally with dose (TABLE 4). Histopathologically, marked bilateral diffuse congestion was noted in the lungs of the animal that died. Arteriopathy, characterized by acute fibrinoid necrosis and hemorrhage of the media, was noted in the coronary arteries; changes were mild and seen primarily in the right coronary arteries. Plasma concentration in this dog 5 minutes postdose was 1960 µg/mL.

**TABLE 4.**

| Plasma CI-1034 Concentrations^{a} | | | | |
|---|---|---|---|---|
| Dose | Plasma Concentration (µg/mL) | | | |
| (mg/kg) | Minutes Postdose | | | |
| | 5 | 15 | 30 | 60 |
| 30 | 301 | 221 | 209 | 157 |
| 40^{b} | 395 | 327 | 267 | 230 |
| 60 | 574 | 427 | 367 | 337 |
| 180^{b,c} | 1960 | -- | -- | -- |

| | | | | |
|---|---|---|---|---|
| ^{a} N=3. | | | | |
| ^{b} N=1. | | | | |
| ^{c} Experiment discontinued approximately 5 minutes postdose; animal subsequently died. | | | | |

### EXAMPLE 10

### Clinical Pharmacology Studies

A total of 48 healthy subjects participated in 3 clinical pharmacology Phase 1 studies that evaluated the tolerance, pharmacokinetics, pharmacodynamics, and drug interaction of CI-1034 (TABLE 5).

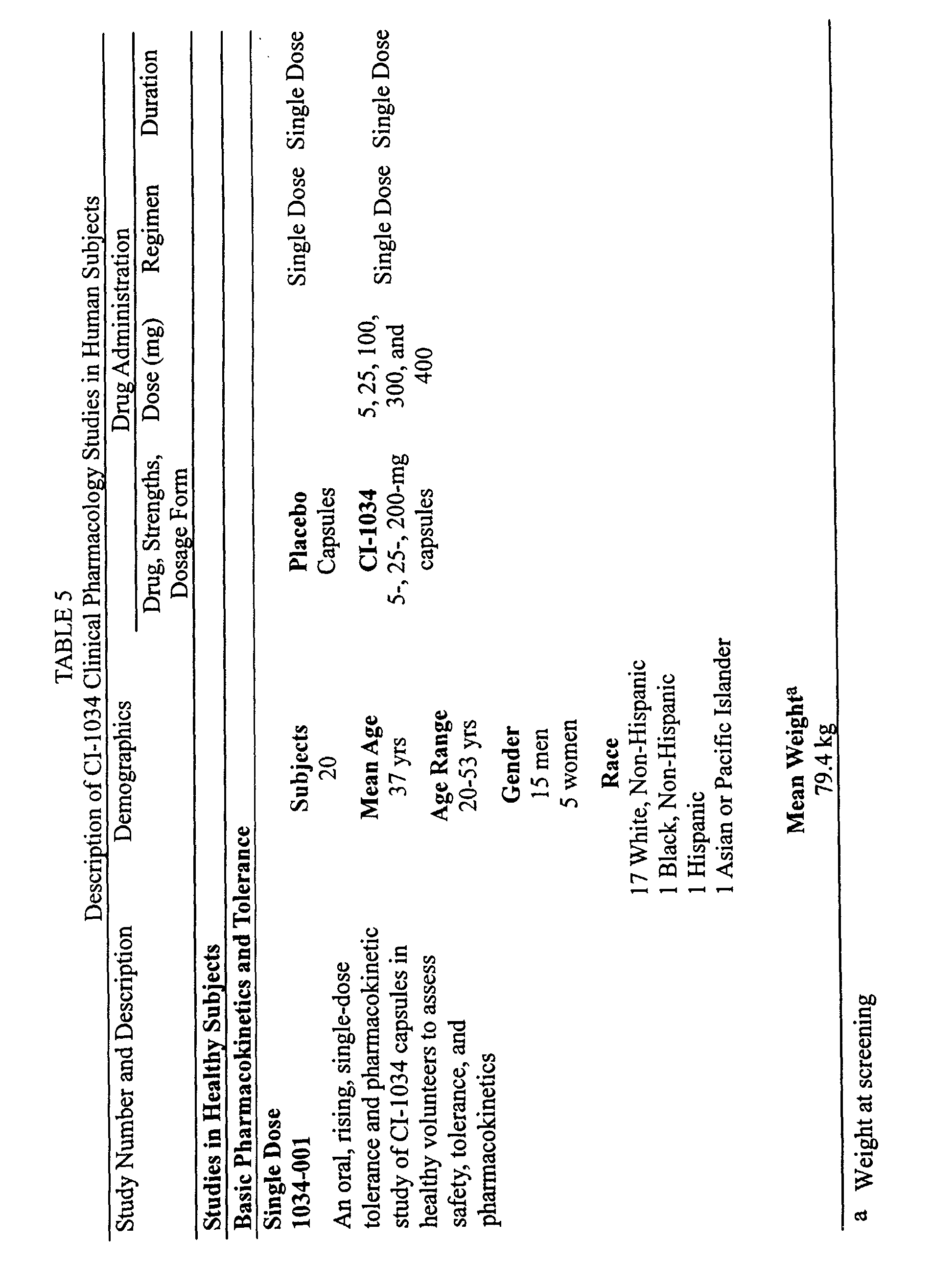

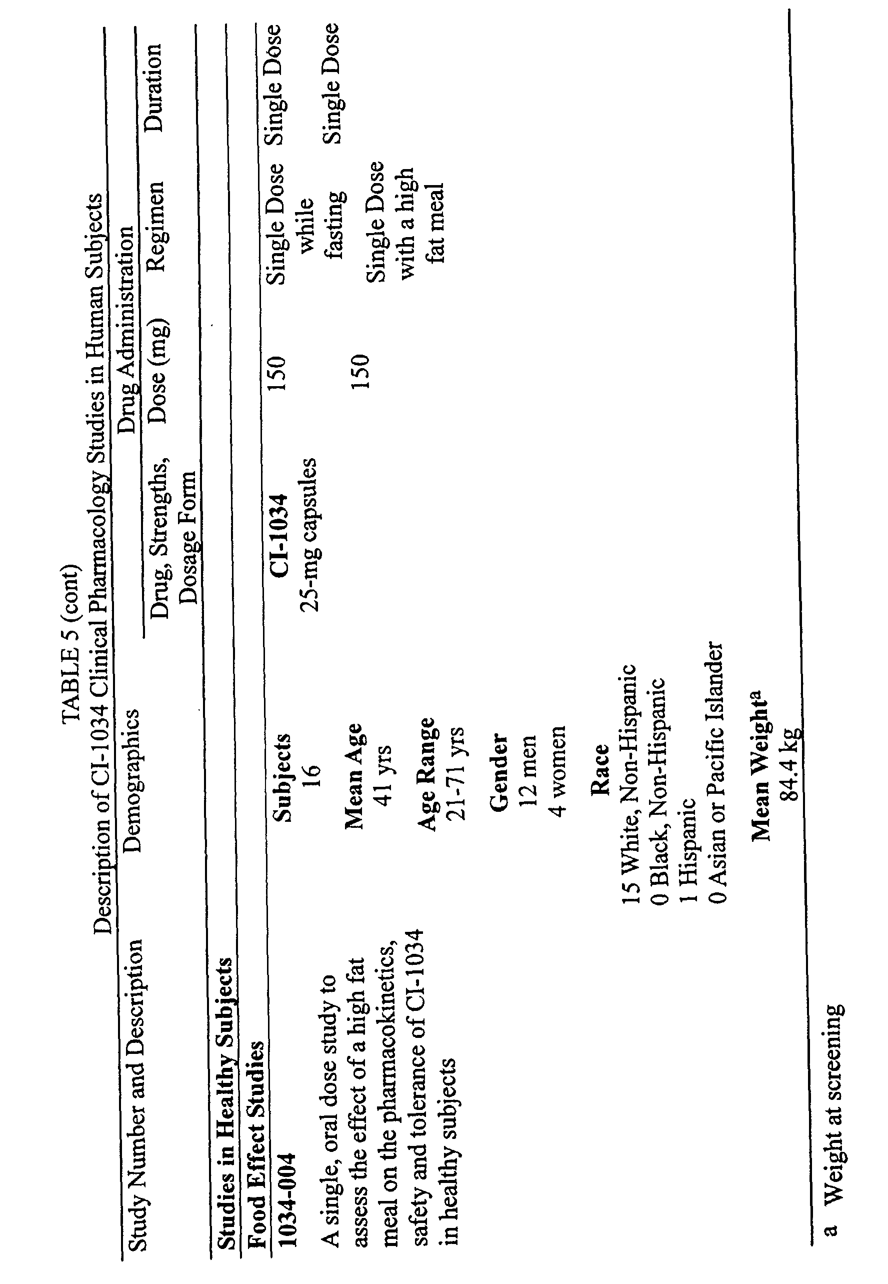

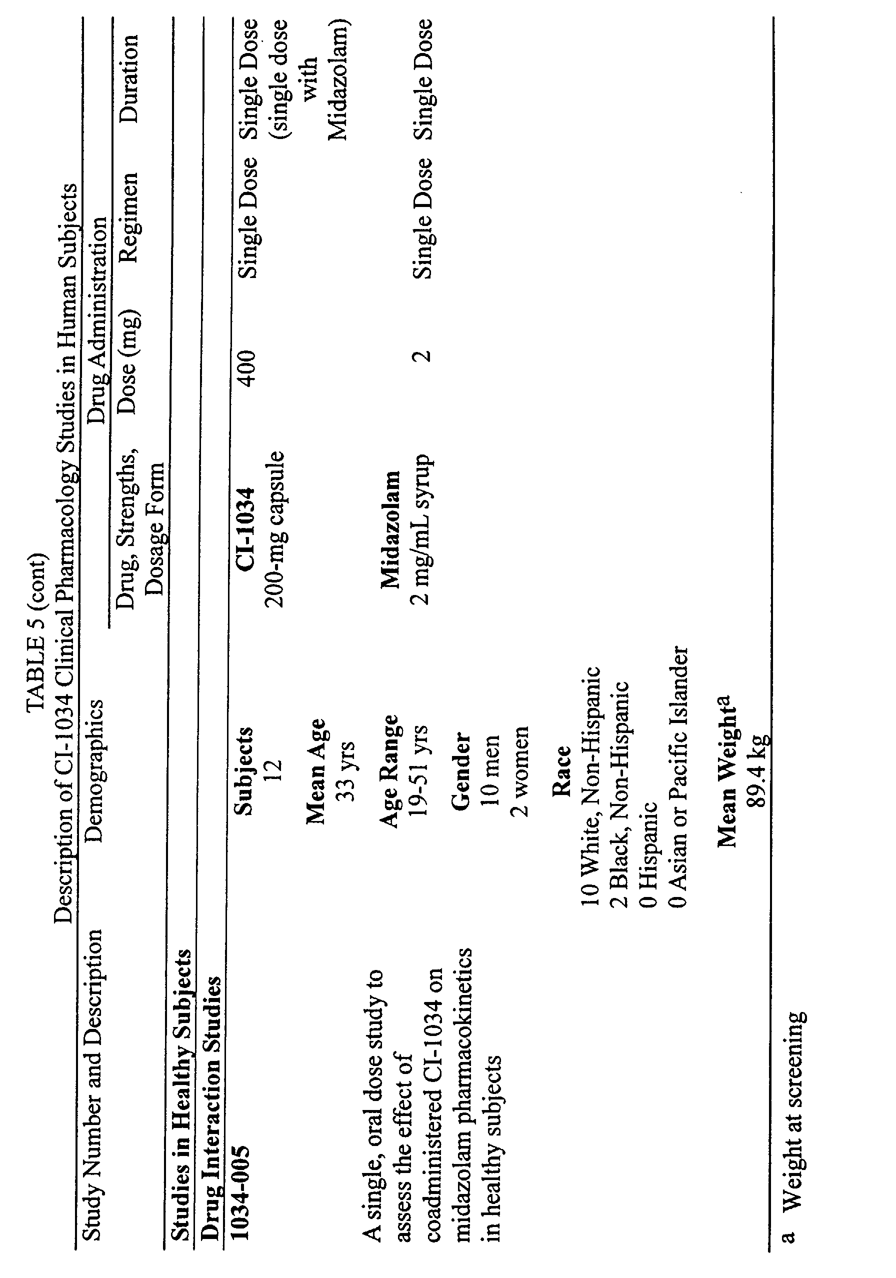

### Pharmacokinetics and Pharmacodynamics

### Single-Dose Study

In Study 1034-001, time to maximal plasma CI-1034 concentrations (tmax) ranged from 1 to 3 hours. The elimination half-life (t½) of CI-1034 ranged from 2 to 4 hours with an additional elimination half-life of approximately 13 hours characterized at the 400-mg dose. Renal excretion of unchanged CI-1034 was <3% of the dose, which is consistent with preclinical observations. There were slightly more than proportional increases in CI-1034 Cmax and AUC(0-∞) values with increasing dose.

Increases in levels of endothelin-1 were observed; however, results were variable and not clearly dose dependent.

### Food Effect on Single Dose of CI-1034

In Study 1034-002 , mean Cmax was approximately 50% lower and mean AUC(0-∞) was approximately 16% lower for CI-1034 administered with a high-fat breakfast when compared to when it was administered during the fasted state. The rate of CI-1034 absorption was slower when administered with a high-fat breakfast compared to when it was administered in the fasted state; tmax increased from 1.26 to 3.5 hours with the addition of the high-fat meal.

### Drug Interaction: Midazolam

In Study 1034-005, coadministration of 2 mg midazolam with 400 mg CI-1034 resulted in an increase in mean plasma midazolam Cmax of 4% (7.65 ng/mL midazolam alone compared with 7.92 ng/mL coadministered). Mean midazolam AUC(0-12) was increased by 43% (15.2 ng·hr/mL alone compared with 21.8 ng·hr/mL when coadministered with 400 mg CI-1034).

### Tolerance and Safety

Single doses of CI-1034 up to 400 mg, and single 150-mg doses of CI-1034 administered with or without a high-fat breakfast were well-tolerated by healthy subjects in single-dose studies 1034-001 and 1034-004, respectively. Concomitant administration of single doses of 400 mg CI-1034 with a single 2-mg dose of midazolam syrup was well-tolerated by healthy subjects in Study 1034-005.

Adverse reactions were usually mild or moderate in intensity and transient. The most frequent adverse events associated with CI-1034 treatment were headache, somnolence, nausea, and dyspepsia. There were no treatment-related serious adverse events. One subject withdrew from the 1034-004 study due to personal reasons unrelated to treatment.

Laboratory abnormalities were generally sporadic and transient and appeared to be unrelated to CI-1034 administration.

No prolonged QTc intervals were observed during the studies.

There were no significant changes in C-reactive protein in healthy subjects in Study 1034-001.

## Claims

1. Use of a selective inhibitor of the ETA receptor for the manufacturing of pharmaceutical compositions for treating chronic obstructive pulmonary disease.

2. Use of 4-(7-ethyl-1,3-benzodioxol-5-yl)-2-[2-(trifluoromethyl)phenyl]-2H-1,2-benzothiazine-3-carboxylic acid 1,1-dioxide or a pharmaceutically acceptable salt thereof for the manufacturing of pharmaceutical compositions for treating chronic obstructive pulmonary disease.

3. Use of 4-(7-ethyl-1,3-benzodioxol-5-yl)-2-[2-(trifluoromethyl)phenyl]-2H-1,2-benzothiazine-3-carboxylic acid 1,1-dioxide monopotassium salt for the manufacturing of pharmaceutical compositions for treating chronic obstructive pulmonary disease.
